# EUROPEAN PATENT SPECIFICATION

(11) **EP 2 091 492 B1**
(45) Date of publication and mention of the grant of the patent: **28.09.2011**
(21) Application number: 06829503.9
(22) Date of filing: 11.12.2006
(51) Int. Cl.: A61F 13/537, A61F 13/513, A61F 13/514, D06M 23/10, D06M 15/227

(54) **ABSORBENT ARTICLE WITH A STRONGLY HYDROPHOBIC LAYER**
SAUGFÄHIGER ARTIKEL MIT STARK HYDROPHOBER SCHICHT
ARTICLE ABSORBANT DOTÉ D'UNE COUCHE FORTEMENT HYDROPHOBE

(43) Date of publication of application: 26.08.2009
(73) Proprietor: SCA Hygiene Products AB, 405 03 Göteborg (SE)
(72) Inventor: BERLAND, Carolyn, 431 36 Mölndal (SE); BITIS, Rozalia, 434 43 Kungsbacka (SE)
(74) Representative: HOFFMANN EITLE
(86) International application number: PCT/EP2006/011915
(87) International publication number: WO 2008/071202

(56) References cited:
- WO-A-00/58410
- WO-A-2004/014575
- WO-A2-02/084013
- ERBIL ET. AL.: "Transformation of a Simple Plastic into a Superhydrophobic Surface" SCIENCE, [Online] vol. 299, no. 5611, 28 February 2003 (2003-02-28), pages 1377-1380, XP002418765 Retrieved from the Internet: URL:http://www.sciencemag.org/cgi/content/ full/299/5611/1377> [retrieved on 2007-02-07] cited in the application

## Description

The present invention relates to an absorbent article such as a diaper, panty diaper, panty liner, sanitary napkin, incontinence device or the like comprising at least one layer made from a strongly hydrophobic material. The high hydrophobicity results from a coating of a hydrophobic thermoplastic polymer having an irregular surface.

### BACKGROUND OF THE INVENTION

Absorbent articles of the present kind generally comprise a liquid permeable coversheet (topsheet) which is located adjacent the wearer's body, a liquid impermeable coversheet (backsheet) which is located distant from the wearer's body and adjacent the wearer's clothing and an absorbent layer interposed between the liquid permeable topsheet and the liquid impermeable backsheet. Sometimes however, in specific absorbent articles, the absorbent layer can also be renounced to.

Over many years diapers have been produced with an outer backsheet formed of a vapour- and liquid-impermeable plastic film to eliminate leakage of fluid from the diaper. However, the feel of plastic backsheets is often perceived by the consumer as cool and unpleasant in comparison to conventional cloth diapers. Moreover, such plastic backsheets also trap the moisture vapour and the heat generated by the body and lead to an environment in the diaper which promotes skin irritation and feels very uncomfortable. Therefore, the majority of absorbent articles such as diapers or sanitary napkins marketed nowadays make use of breathable backsheet materials which permit vapours to escape from the absorbent article while still preventing exudates from passing through the backsheet. Exemplary breathable materials are for instance woven webs, nonwoven webs, composite materials such as film-coated nonwoven webs and microporous films. Even with modern backsheet materials, there is still a trade off between the capacity of a backsheet material to prevent the passage of body liquids and its breathability. Therefore, it would be desirable to increase the hydrophobicity of backsheet materials since highly hydrophobic materials could be imparted with a higher breathability (for instance by increasing the number and/or size of pores) without impairing the liquid-impermeability.

Another problem occurring in diapers and sanitary napkins is the uneven distribution of body liquid in the absorbent layer. The body fluid insulting into the central area of a disposable absorbent product tends to wet only the centre of the absorbent layer while the edges, especially in the elongated direction remain dry. Accordingly, the absorbing capacity of the absorbent layer is not used effectively. Moreover, so-called "gel blocking" may occur since the superabsorbent polymer often used in the absorbent layer swells in the central area under formation of a gel and prevents further spreading of the fluid. Therefore, current absorbent articles, in particular current diapers typically possess so-called fluid handling layers interposed between the topsheet and the absorbent layer. These fluid handling layer enhance fluid transport in a plane parallel to the absorbent layer, preferably in the elongated direction of the absorbent product. This may for instance be achieved by a nonwoven sheet material containing oriented hydrophilic fibers. Furthermore, EP 0 748 894 A2 discloses in the same connection a method for increasing directionality of fluid transport in nonwoven sheet materials, for instance by printing hydrophilic nonwovens with elongated, continuous hydrophobic stripes. The hydrophobizing agents are preferably water-insoluble paraffin compounds. The hydrophobic stripes are said to have a water contact angle over 90°. The resulting nonwoven materials can be used as cover stock material (fluid permeable topsheet), as sublayers or transport layers (fluid handling layers) in disposable, hygienic absorbent articles. In view of this teaching it would be desirable to achieve an even stronger increase in directionality of fluid transport.

EP 0 985 392 A1 relates to a disposable absorbent article comprising at least one "superhydrophobic" layer having a static water contact angle higher than about 120°, most preferably between 150 and 165°. In the examples small silicone, PE (polyethylene) or PP (polypropylene) substrates are subjected to a modulated glow discharge plasma treatment performed with,a fluorocarbon gas or vapour to generate a continuous, fluorocarbon thin film with a superhydrophobic surface tightly bound to the substrate. Allegedly, this technique can also be applied to other substrates including nonwoven layers. Apart from the fact that experimental proof is missing as to whether this coating technique can be successfully applied to nonwovens it remains to be noted that this coating technique is cumbersome and costly. Moreover, for environmental reasons, it is undesirable to use a fluorocarbon coating in a disposable absorbent hygiene article.

WO 02/084013 A2 discloses a polymer fiber having a self-cleaning and water repellent surface which is comprised of at least one synthetic fiber material and synthetic, at least partially hydrophobic surface with elevations and depressions made of particles that are joined to the fiber material without adhesive, resins or lacquers. The only example discloses a polyamide fiber having an advancing contact angle of almost 160°. It is also stated that these fibers can be used in various areas, mostly for sportive activities. Hints to the use of these fibers in nonwoven materials or absorbent hygiene articles of the claimed type are missing.

US 2002/0013560 discloses a unitary absorbent core including a fibrous absorbent layer having an upper fluid receiving surface and a lower surface with a hydrophobic vapour-transmissive moisture barrier integral with the lower surface of the absorbent layer. Also disclosed is a process wherein to the lower surface of the fibrous absorbent layer a hydrophobic material is applied which at least partially coats at least some of the fibers of the lower surface of the absorbent layer. In a preferred embodiment, the vapour-transmissive moisture barrier is formed by applying a hydrophobic polymeric latex emulsion to achieve a contact angle for water to about 80° or greater.

WO 90/05063 concerns a wrap composed of paper, paper board of similar fibrous material which is coated with a vapour barrier type of coating mix containing polyolefin plastic. As typical application for such wraps this document mentions the wrapping of large-size paper rolls. The coating mix is composed of isotactic polypropylene with atactic polypropylene or amorphous poly-α-olefin acting as a plasticizer.

WO 97/16148 relates to a breathable diaper, feminine hygiene or like disposable sanitary product including a backsheet formed of a multilayer nonwoven material which is hydrophobic and vapour permeable, said backsheet material having at least two melt-blown layers. The construction preferably includes a hydrophobic enhancer formed of a multilayer nonwoven material. The hydrophobic enhancer is disposed at least partially outwardly of the barrier base and inwardly of the backsheet. The hydrophobic enhancer may be a hydrophobic coating disposed adjacent an inner surface of the backsheet, the coating being polymeric, but cracked or fractured to provide breathability thereto. The cracked coating is preferably an ethyl-vinylacetate (EVA) extrusion having cracks or fractures sufficient to provide breathability thereto.

US 2005/0004541 A1 is directed to an absorbent core which may be used with food packaging to absorb and retain fluid exuded by a food product. This absorbent core includes a first fibrous absorbent layer the lower surface of which is in contact with an upper surface of a synthetic carrier which has a lower surface integral with a first hydrophobic vapour transmissive moisture barrier. The hydrophobic barrier material coats at least some of the individual fibers of the absorbent layer. In a preferred embodiment the vapour transmissive moisture barrier is formed by applying a hydrophobic polymeric latex emulsion to achieve a contact angle for water of about 80° or greater.

SE 8,502,556 concerns a method of applying synthetic polymers, for examples polyolefins on fibers to facilitate thermal consolidation of fiber mats by dry forming. In example 1 a solution of polypropylene in xylene at a concentration of 1 to 2% was prepared. Dried and finely separated pulp material was added to this solution to obtain a fiber concentration of 2% and the mixture was heated to a temperature of 100 to 115°C. After about 15 min the pulp material was removed from the solution, pressed to obtain a dry content of about 20% and thereafter dried.

WO 2006/049664 discloses a composite material comprising a plurality of nanofibers intertwined with a plurality of cores fibers such as pulp fibers to form one or more layers. The resulting web materials can be used in absorbent products. In one embodiment, some or all of the nanofibers comprise hydrophobic fibers of sufficiently small diameter to simulate the lotus effect in their hydrophobicity and self-cleaning abilities. According to this document the nanofibers having an average diameter not greater than about 1500 nm and are produced by electrospinning. For some applications it is however not favourable to incorporate such fine fibers into a web material. Furthermore, electrospinning represents an expensive and complicated manufacturing technique.

WO 2005/005696 relates to a nonwoven web comprising a layer having a significant number of nanofibers with diameters less than 1 µm, wherein a coating substance is applied to a surface of said nanofibers. One among many examples of coating substances are hydrophobic treatments such as poly-di-methylsiloxane. The resulting nonwoven webs may be utilized as barrier layer disposed between an absorbent core and an outer layer of a disposable absorbent product. However, this document does neither relate to the coating with thermoplastic polymers nor the formation of an irregular surface (rough surface) enhancing hydrophobicity.

US 2006/0094320 A1 has a similar disclosure than WO 2006/049664 A1.

Other applications relating to nanofiber-based webs and their use as barrier layers are WO 2005/103354, WO 2005/103357, WO 2005/004769, WO 2005/005704, WO 2005/004767, US 2005/0008776 and US 2004/0266300.

WO 2004/014575 A1 relates to a method for producing self-cleaning surfaces by means of dry coating methods. According to the method, self-cleaning surfaces with elevations are produced. Said elevations are formed by particles which are applied in a dry state to the surface by an electrostatic powder spraying method. According to said method, textiles and other objects, especially those having plastic surfaces, can be made self-cleaning.

The particles can be selected from silicates, minerals, metal oxides, silicic acid, pigments or polymers. As one embodiment of polymers PTFE powder is mentioned. Products and applications wherein the resulting lotus surfaces can be used include textiles, films, three-dimensional items, membranes, textiles/non-wovens, van covers, hygiene non-wovens, diapers, household devices, devices for outdoor use, sport goods, devices for sailing and motor sports, tents, sun covers, umbrellas, etc.

US 4,808,474 relates according to claim 1 to a disposable diaper having attached thereto as a closure means a pressure-sensitive adhesive tape comprising a backing film made from a blend of crystalline isotactic polypropylene and a compatible polymer, said film fulfilling certain mechanical requirements. According to the experimental section (column 8, lines 6-60), a polymer blend comprising isotactic polypropylene is heated and extruded through a slot extrusion die and then into the nip between a silicone rubber-covered roll having an average roughness (Ra) of 20, and an average maximum profile height (Rpm) of 115, and a water-cooled metal chill roll having an Ra of 250 and an Rpm of 220 to effect quenching and provide a matte finish.

Accordingly, the present invention aims at providing an absorbent article with improved properties in a simple manufacturing process.

The present invention also aims at overcoming drawbacks associated with the prior art.

The technical object of the present invention also involves the aspect of modifying layers, in particular web, foam or film materials used in absorbent articles in a manner leading to a drier environment and thus more skin comfort, for instance by directing fluid transport and/or modifying the surface properties of said layers in a manner that allows for using materials having a higher breathability.

The present invention also aims at providing a simple method for producing such absorbent articles.

Further technical objects will become apparent from the preceding discussion of the prior art and the following more detailed description of the present invention.

### SUMMARY OF THE PRESENT INVENTION

The present invention provides
- an absorbent article such as a diaper, panty diaper, panty liner, a sanitary napkin, an incontinence device or the like, comprising at least a liquid-permeable topsheet made from at least one layer of web, foam or film material and a (preferably liquid-impermeable) backsheet made from at least one layer of web, foam or film material, and optionally at least one further layer of web, foam or film material,
   wherein at least one of said layers comprises a coating of a hydrophobic thermoplastic polymer having an irregular surface as defined in claim 1 and shows a contact angle of a sessile water drop of more than 110°; and
- a method of making this absorbent article, an absorbent article such as a diaper, panty diaper, panty liner, a sanitary napkin, an incontinence device or the like, comprising at least a liquid-permeable topsheet made from at least one layer of web, foam or film material and a (preferably liquid-impermeable) backsheet made from at least one layer of web, foam or film material, and optionally at least one further layer of web, foam or film material,
   wherein at least one of said layers comprises a coating of a hydrophobic thermoplastic polymer having an irregular surface and shows a contact angle of a sessile water drop of more than 110°, said method comprising the steps of
   applying onto at least one of said layers a solution of a hydrophobic thermoplastic polymer in an organic solvent and evaporating said solvent to form a coating having an irregular surface, and incorporating said coated layer into the absorbent article.

### FIGURES

Figures 1 (A) and (B) show ESEM pictures of wipe material coated with isotactic propylene in accordance with the present invention. The uncoated substrate is shown in Figure 1 (C).
Figure 2 shows schematically a test device used for evaluating water-impermeability of coated nonwoven materials via hydrostatic pressure.
Figure 3 shows an schematically arrangement of two glass plates used by the present inventors to fix nonwovens to be coated.

### DETAILED DESCRIPTION OF THE PRESENT INVENTION

Wherever the following description uses the term "comprising" or "containing" it shall be understood that the same may also be replaced by the more restrictive terms "essentially consisting of" and "consisting of" as long as this leads to technically meaningful embodiments.

As "absorbent article" we understand articles capable of absorbing body fluids such as urine, watery feces, female secretion or menstrual fluids. These absorbent articles include, but are not limited to diapers, panty diapers, panty liners, sanitary napkins or incontinence devices (as for instance used for adults).

Such absorbent articles have a liquid-permeable coversheet (topsheet) which during use is facing the wearer's body. They further comprise a (preferably liquid-impermeable) coversheet (backsheet), for instance a plastic film, a plastic-coated nonwoven or a hydrophobic nonwoven and preferably an absorbent layer enclosed between the liquid-permeable topsheet and the preferably liquid-impermeable backsheet. In some absorbent products without absorbent layer, such as specific panty liners marketed by the present applicant under various trademarks in connection with the product name "Freshness everyday", the absorbent capacity of topsheet and backsheet is sufficient to absorb small amounts of female secretion.

"Liquid-impermeable" materials, such as liquid-impermeable nonwoven and film materials (e.g. microporous film) are capable of preventing body liquids such as urine, menses and watery feces from passing through under normal conditions of use. They are preferably employed as backsheet and leg cuff material. According to preferred embodiments, the liquid-impermeability of such materials can be expressed as water resistance according to EDANA WSP 80.6 (05) (see also examples) of more than 40 mbar, more than 50 mbar, more than 55 mbar, more than 60 mbar, more than 65 mbar, more than 70 mbar, more than 75 mbar, or more than 80 mbar, with increasing preference, e.g. as water resistance of more than 85 mbar to 150 mbar, or 90 to 120 mbar. If not stated otherwise, this relates to all liquid-impermeable materials mentioned in this application.

In absorbent articles of the claimed type at least one layer of web, foam or film material comprises a coating of the hydrophobic thermoplastic polymer, said coating having an irregular surface. The term "irregular surface" is intended to cover surface structures increasing the hydrophobicity beyond the hydrophobicity of a smooth surface made from the thermoplastic polymer constituting the coating. This effect also known as "lotus effect" typically arises in the presence of micrometer roughness. The term "irregular surface" also distinguishes the present invention from uniform coatings of thermoplastic polymers on layer materials of the described kind where the thickness of the coating essentially does not change over the coated substrate (film, foam or fibers of web). The irregular surface comprises, or preferably consists of, individual deposits (e.g. essentially spherical deposits or thread-like deposits) of coating material which may also aggregate and/or of a network structure consisting of interconnected deposits forming (air-filled) pores. Preferably, at least some (e.g. at least 10%, at least 30% or at least 50%) of the observable dimensions, more preferably the average peak-to-valley height for aggregates, the pore diameter for networks or the diameter of thread-like deposits have an average size of at least 1 µm, in particular at least 3 µm, but preferably not more than 100 µm, e.g. 5 to 50 µm. According to an alternative embodiment, a standard roughness measure such as the arithmetic average roughness Ra is at least 1 µm, in particular at least 3 µm, but preferably not more than 100 µm, e.g. 5 to 50 µm. The roughness measured shall be the roughness of the coating, not the roughness of the underlying structure such as nonwoven. Measurement can be made on the surface of a single film.

The average peak-to-valley-height is measured in respect of the coated surface, e.g. film or foam surface or fiber surface in the case of webs, by means of optical measurement methods such as SEM techniques, profiling techniques such as AFM stylus profilers. Alternatively optical techniques can be used. Preferably, the determination of the above concrete roughness values is conducted using SEM, optionally in connection with image processing software.

The coated layer of web, foam or film material shows contact angle values of a water drop contacting said layer of more than 110°, preferably more than 120°, even more preferably more than 130°, for instance more than 140° or more than 150°. One technique for achieving this high degree of hydrophobicity will be explained in further detail in connection with the claimed method. It is based on a procedure described for the first time by H. Ü. Erbil et al., Science, Vol. 299, 2003, pages 1377-1380, "Transformation of a simple plastic into a superhydrophobic surface". In accordance with the present invention and the Erbil reference such superhydrophobic surfaces result from the increase of the surface roughness so that the local geometry provides a large geometric area for a relatively small projected area. This effect can be observed in nature on the leaves of the sacred lotus. The surfaces of these leaves have micrometer-scale roughness (as also observed for the present invention) resulting in water contact angles up to 170°. In the present invention air that is trapped between the droplets and the irregular surface equally minimizes the contact area.

The contact angle can be determined in line with TAPPI method T558PM-95 (1995) according to a procedure described in the examples.

If the coating of thermoplastic polymer only partially covers the web, foam or film material on a macroscopic scale the above contact angle measurement is conducted solely in respect of the coated area.

The "**film material**" to be coated can be any polymer film material usable in absorbent articles. It is preferably a (macro-)perforated plastic films (as used for topsheets) or a microporous breathable film as commonly used as backsheet material. Suitable microporous films will be explained later in further detail in connection with embodiments relating to hydrophobic coatings on the backsheet and parts thereof.

As **"web material"** we understand preferably coherent flat fiber-based structures, in particular of paper tissue, woven or nonwoven type.

A tissue paper is defined as a soft absorbent paper having a low basis weight. One generally selects a basis weight of 8 to 30 g/m², especially 10 to 25 g/m² per tissue layer. Each tissue layer may consist of various non-separable sublayers generated for instance by means of a multiple headbox in the paper machine. The density of tissue is typically below 0.6 g/cm³, preferably below 0.30 g/cm³ and more preferably between 0.08 and 0.20 g/cm³.

The production of tissue is distinguished from paper production by its extremely low basis weight and its much higher tensile energy absorption index (see DIN EN 12625-4 and DIN EN 12625-5). Paper and tissue paper also differ in general with regard to the modulus of elasticity that characterizes the stress-strain properties of these planar products as a material parameter.

A tissue's high tensile energy absorption index results from the outer or inner creping. The former is produced by compression of the paper web adhering to a dry cylinder as a result of the action of a crepe doctor or in the latter instance as a result of a difference in speed between two wires ("fabrics"). This causes the still moist, plastically deformable paper web to be internally broken up by compression and shearing, thereby rendering it more stretchable under load than an uncreped paper.

Moist tissue paper webs are usually dried by the so-called Yankee drying, the through air drying (TAD) or the impulse drying method.

The fibers contained in the tissue paper are mainly cellulosic fibres, such as pulp fibers from chemical pulp (e.g. Kraft sulfite and sulfate pulps), mechanical pulp (e.g. ground wood), thermo mechanical pulp, chemo-mechanical pulp and/or chemo-thermo mechanical pulp (CTMP). Pulps derived from both deciduous (hardwood) and coniferous (softwood) can be used.

Nonwovens represent flexible porous fabrics, which frequently resemble textiles, but are not produced by the classic methods of weaving warp and weft or by looping, but by intertwining and/or by cohesive and/or adhesive bonding of typical synthetic textile fibers which may for example be present in the form of endless threads or threads prefabricated with an endless length, as synthetic threads produced *in situ* or in the form of staple fibers. Alternatively, they may be made of blends of synthetic fibers in the form of staple fibers and natural fibers, e.g. natural vegetable fibers (see DIN 61 210 T2 of October 1988 and ISO 9092 - EN 29092). Further embodiments will be explained in connection with topsheet materials.

According to the present invention, the coating of the **hydrophobic thermoplastic polymer** comprises crystalline domains. For this reason, it is less desirable to employ amorphous thermoplastic polymers since these lack the capability to form such crystalline domains. Accordingly, it is preferred to utilize as starting material for the coating semicrystalline and crystalline thermoplastic polymers. The degree of crystallinity (prior to the formation of the coating) is preferably at least 30, 40, 50, 60, 70, 80, 90% with increasing preference in this order. The degree of crystallinity can be measured according to methods known in the art, for instance by X-ray diffraction analysis. As will be explained later in further detail, it is believed that the crystalline or semi-crystalline nature of the thermoplastic starting polymer, and the capacity to form crystalline domains during rapid cooling, enhances the formation of an irregular surface and thereby strongly increases hydrophobicity of the coating. During this rapid cooling from a preferably hot solution of the thermoplastic polymer the degree of crystallinity may lower depending on the deposition rate. The degree of crystallinity in the resulting coating may thus be more than 20%, more preferably more than 30%, even more preferably more than 40%, in particular more than 50%, for instance 60 to 100% or 70 to 90%.

The hydrophobic thermoplastic polymer is preferably constituted by monomers consisting of carbon and hydrogen atoms. Although it is conceivable to use, at least in a minor proportion, monomers containing also other atoms such as N or O, this is less preferred. Similarly, the thermoplastic polymer is preferably free of fluorine atoms. The thermoplastic polymer is preferably a polyolefin homo- or copolymer. Examples for polyolefin homopolymers are polyethylene and polypropylene. Ethylene and/or propylene may also be copolymerized with other ethylenically unsaturated monomers as long as the resulting copolymer still can be regarded as thermoplastic. Ethylene/propylene copolymers may also be used. To lower the melting point propene may for instance be copolymerized with minor amounts (e.g. less than 10 wt.-%) of another α-olefin such as ethylene, 1-butene or 1-hexene. Preferably, the comonomer and its amount are selected having regard to the desired degree of crystallinity.

One particularly preferred thermoplastic polymer is isotactic polypropylene. Depending on the catalyst system used for its manufacture its isotactic index (% insoluble in boiling heptane) is preferably at least 88%, more preferably at least 92%, in particular at least 98% by weight (see Ullmann's Encyclopedia of Industrial Chemistry, fifth completely revised edition, volume A 21, 1992, pages 518 to 547). The isotactic polypropylene is preferably obtained from the polymerization of propene in the presence of heterogeneous Ziegler-Natta catalysts. With the more recent catalyst generation (MgCl₂-supported TiCl₄ x AlEt₃) an isotactic index of more than 98 wt.-% is for instance achievable. The melt flow index (230°C/2.16 kg) of the thermoplastic polymer ranges preferably from 0.3 to 50 dg/min, e.g. from 1 to 40, or 5 to 10. Typical and preferred Mw/Mn values are 5 to 10. In terms of melting point the thermoplastic polymer is also not subject to a particular restriction. Typical melting points are in the range from 130 to 200°C, for instance 150 to 190°C. One suitable and commercially available thermoplastic polymer is isotactic polypropylene which is offered by Sigma-Aldrich Co. under the product number 182389 (average Mw - 250000 GPC, melting point 189°C).

Depending on the substrate material (layer of web, foam or film material) to be coated it may also be preferred to generate a **non-sealing coating.** The term "non-sealing" denotes coatings where the coating does not cover the entire coated area and correspondingly still allows for the passage air and/or water vapour. The term "non-sealing" is however not restricted to certain shapes of the coating and passageways leading from one side of the coating through the coating to the other side. As explained before, the non-sealing coating preferably comprises individual deposits of thermoplastic polymer which may however form larger aggregates (e.g. essentially spherical deposits or thread-like deposits) and/or of a network structure consisting of interconnected deposits forming (air-filled) pores.

The use of non-sealing coatings is particularly preferred in combination with layer materials which are **air- and/or water vapor-permeable** (breathable) themselves such as foam, nonwoven, tissue paper, perforated plastic films (as used for topsheets) or microporous plastic films (as used for backsheets). Wherever the present application refers to "breathable" materials it is preferred that the same display with increasing preference WVTR values (water vapor transmission rate) of more than 600, more than 900, more than 1200, more than 1500, more than 1800, more than 2100, more than 2400, more than 2700, more than 3000, more than 3300, more than 3600 g/m² x 24h measured according to EDANA WSP 70.6 (05) part 2 as specified in the examples, e.g. 4200 to 6000, or 4500 to 4800 g/m² x 24h.

Especially with a (preferably) liquid-impermeable but vapor-permeable (breathable) material it is strongly preferred to apply the coating of thermoplastic polymer in an amount and a manner that reduces the inherent breathability of the material as little as possible. In one alternative and preferred embodiment of the invention, it is also possible to render common liquid-permeable nonwovens (of the type described herein) liquid-impermeable by coating these with thermoplastic polymer to generate an irregular and strongly hydrophobic surface. The resulting material will still show the necessary breathability but can be used as backsheet material owing to its capacity to prevent the passage of body liquids. Nonwovens treated in this manner can also be used as leg cuffs as will be explained later.

If, on the other hand, the liquid permeability of the substrate to be treated (layer of web, foam or film material) is essential for its functioning in the absorbent article typically only a partial coating is applied thereto, for instance a regular pattern of hydrophobic areas with thermoplastic polymer that guide the liquid flow in a certain direction.

Preferably, the non-sealing coating of thermoplastic polymer comprises individual, and discretely discernible deposits (e.g. essentially spherical deposits or thread-like deposits) of coating material which may also aggregate and/or a network structure consisting of interconnected deposits forming (air-filled) pores. The network structure may be constituted by branched and intermingled sticks and bumps of thermoplastic polymer as described by Erbil. The network may also be described as a sponge-like coherent deposition of thermoplastic polymer.

Network-like structures typically arise when the substrate material is dipped into a solution of the thermoplastic polymer or such solution is cast onto the substrate prior to a relatively quick evaporation of the organic solvent. The spray deposition of the thermoplastic polymer solution on the substrate, on the other hand, seems to enhance the formation of the afore-mentioned essentially spherical deposits of thermoplastic polymer, preferably isotactic polypropylene. Preferably, these spherical deposits possess each a rough and structured surface and/or form large aggregates randomly spread over the substrate. The individual spherical deposits may for instance show a diameter in the range of 5 to 50 µm, e.g. 10 to 20 µm.

If the non-sealing coating comprises pores, the majority of pores visible at the surface preferably has a size below 100 µm, more preferably below 50 µm, for instance below 40 or below 30 µm. In this connection "majority of pores" means more than 50% while a ratio of at least 60%, at least 70% or at least 80% is preferred.

The strongly hydrophobic coating having an irregular surface is preferably obtainable by contacting the layer (web, foam or film) to be treated with a preferably hot solution of the thermoplastic polymer in a suitable organic solvent followed by evaporating said solvent. Details of more preferred embodiments of this procedure will be explained later in connection with the claimed method.

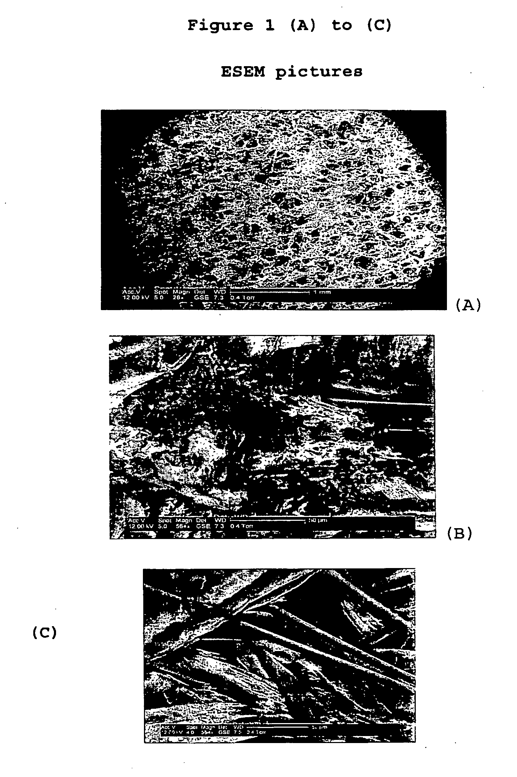

According to one embodiment of the present invention, the **liquid permeable coversheet** (**topsheet**) comprises the coating of a hydrophobic thermoplastic polymer. Moreover, it is preferred that this coating does not cover the entire topsheet surface. Preferably, the coating is applied in a pattern, in particular in a regular pattern. In this embodiment the coating preferably constitutes from 5 to 90%, more preferably from 10 to 80%, in particular from 20 to 70% (e.g. from 30 to 60%) of the entire topsheet area. According to one embodiment, the pattern comprises parallel stripes running in the longitudinal direction of the coversheet. These stripes may for instance be elongated continuous hydrophobic stripes of the type shown in figure 1 of EP 0 748 894 A2. The width of these stripes is not particularly restricted. For practical reasons it lies usually in the range of from 0.2 to 2 cm, in particular 0.5 to 1.5 cm.

The above-explained partial coating, in particular pattern of hydrophobic thermoplastic polymer is preferably applied to hydrophilic topsheet materials. As "**hydrophilic**" we do not understand solely those topsheet and other materials that were made from hydrophilic fibers or polymers such as cellulosic fibers, e.g. cotton, pulp, rayon or viscose fibers, or polyester, polyamide (e.g. nylon), acrylic (e.g. polyacrylonitrile) polymers or polyurethane or wool fibers. The term "hydrophilic" equally extends to topsheet and other materials, for instance nonwoven materials that were made from hydrophobic fibers or materials and subjected to a hydrophilizing treatment, for instance with a suitable surfactant and/or a physical hydrophilizing treatment such as corona discharge, plasma or flame treatment. Generally, "hydrophilic" materials are characterized by contact angles of a sessile water drop on a smooth surface of less than 90° while "**hydrophobic**" materials show contact angles of a sessile water drop on a smooth surface thereof of more than 90°.

A suitable **topsheet** may be manufactured from a wide range of materials such as woven and nonwoven materials (e.g. a nonwoven web of fibers), polymeric materials such as apertured plastic films, e.g. apertured formed thermoplastic films and hydroformed thermoplastic films; porous foams; reticulated foams; reticulated thermoplastic films; and thermoplastic scrims. Suitable woven and nonwoven materials can be comprised of natural fibers or from a combination of natural and synthetic fibers. Examples of suitable synthetic fibers which may comprise all or part of the topsheet include but are not limited to polyamide (e.g. nylon), acrylic (e.g. polyacrylonitrile), aromatic polyamide (e.g. aramide), polyolefin (e.g. polyethylene and polypropylene), polyester, butadiene-styrene block copolymers, natural rubber, latex, spandex (polyurethane) and combinations thereof. Synthetic fibers that contain more than one type of repeat unit can result from combining repeat units at the molecular level within each macromolecular strand (copolymer), between macromolecular strands (homopolymer blends), or combinations thereof (co-polymer blends); or they can result from combining repeat units at a higher scale level with distinct nanoscopic, microscopic, or macroscopic phases (e.g., multicomponent fibers). Each component of a multicomponent fiber can comprise a homopolymer, a copolymer, or blends thereof. Bicomponent fibers are common versions of multicomponent fibers. The two or more types of repeat units in a copolymer can be arranged randomly or in alternating blocks of each type. Blocks of different types of repeat units can jointed to one another at their respective ends (block co-polymers) or between the respective end of at least one block (graft co-polymers).

Nonwoven materials can be formed by direct extrusion processes during which the fibers and the nonwoven materials are formed at about the same point in time, or by preformed fibers which can be laid into nonwoven materials at a distinctly subsequent point in time. Exemplary direct extrusion processes include but are not limited to: spunbonding, meltblowing, solvent spinning, electrospinning and combinations thereof typically forming layers. Exemplary "laying" processes including wet laying and dry laying. Exemplary dry laying processes include but are not limited to air laying, carding and combinations thereof typically forming layers. Combinations of the above processes yield nonwovens commonly called hybrides or composites.

The fibers in a nonwoven material are typically joined to one or more adjacent fibers at some of the overlapping junctions. This includes joining fibers within each layer and joining fibers between layers when there is more than one layer. Fibers can be joined by mechanical entanglement, by chemical bond or by combinations thereof. A more detailed description of suitable topsheet materials which can be applied to the present invention and is incorporated by reference is found in US 2004/0158214 A1, specifically in the passage from par. [0043] to [0051].

In accordance with the invention, it is preferred to make use of apertured plastic films (e.g. thermoplastic films) or nonwoven materials based on synthetic fibers, e.g. those made from polyethylene or polypropylene homo- or copolymers and polymer compositions based thereon.

Examples for nonwovens made from hydrophilic materials (block copolymers) or being treated with durable hydrophilizing agents are found in EP 0 597 224 A, WO 94/28838, EP 0 539 703 A, EP 0 598 204 A, WO 95/10648, EP 0 340 763 A, WO 98/10724, and EP 0 516 271 A.

Further, it is preferred that the nonwoven materials to be coated according to the present invention are made from fibers having a diameter above the nm range, i.e. above 1µm, preferably above 2pm. In other words, preferred nonwovens do not comprise nanofibers.

According to one further embodiment, the absorbent article is a diaper, panty diaper, incontinence device or the like absorbent article and comprises (as the optional "at least one further layer") **leg cuffs** carrying the coating of a hydrophobic thermoplastic polymer. This coating is effective in preventing body liquids from leaking through the leg cuffs. Simultaneously, by virtue of the resulting higher liquid-impermeability, said leg cuffs can be provided with greater breathability which is beneficial to the climate in the absorbent article.

This embodiment solves the objects of the present invention irrespective of the material used for the topsheet and backsheet. Moreover, absorbent articles comprising leg cuffs typically employ an absorbent layer made from one or more absorbent materials as explained below in further detail. Accordingly, this embodiment can also be described as absorbent article comprising at least a liquid-permeable topsheet preferably made from at least one layer of web, foam or film material and a backsheet preferably made from at least one layer of web, foam or film material, an absorbent layer (core) enclosed between said topsheet and backsheet, and leg cuffs preferably disposed adjacent to the two (longitudinal) edges of the absorbent article as shown for instance in US 4,695,278,
wherein said leg cuffs comprise a coating of a hydrophobic thermoplastic polymer having an irregular surface and show a contact angle of a sessile water drop of more than 110°.

Generally, various manners of performing this embodiment of the invention are conceivable while in each case the leg cuffs are preferably made from nonwovens and the resulting coated nonwoven material is still considered to be breathable:
- (A) Hydrophilic nonwovens of the type described before are fully coated on at least on side, preferably both sides with the hydrophobic thermoplastic polymer to render them liquid-impermeable, but still breathable. Both properties are reflected by water pillar (in mbar) and WVTR values as disclosed before. The nonwoven to be coated contains at least some hydrophilic fibers, for instance at least 50% by weight, for instance at least 70% or at least 80% by weight of hydrophilic fibers. According to one further embodiment the hydrophilic nonwoven fully consists of hydrophilic fibers. The hydrophilic fibers are preferably selected from cellulosic fibers, such as viscose, rayon, cotton, wood pulp fibers, polyester or polyamide such as nylon fibers. It is preferred to use a precoated material, that is a material coated by nonwoven manufacturers and supplied as roll.
- (B) Hydrophobic liquid-impermeable nonwoven materials as currently used for leg cuffs (having for instance a water resistance according to EDANA WSP 80.6 (05) (see so examples) of more than 30 mbar, more than 35 mbar, or more than 40 mbar) are coated on at least on side, preferably on both sides with the hydrophobic thermoplastic polymer to further enhance their liquid impermeability, for instance by at least 10, at least 20 or at least 30 mbar.
- (C) Rather lofty nonwoven materials which may not yet have the desired liquid-impermeability (as reflected for instance by a water resistance of less than 30 mbar in accordance with Edana WSP 80.6 (05) as specified in the examples) are coated on at least one side, preferably both sides with the thermoplastic polymer to achieve an excellent balance of liquid impermeability and high breathability. Preferably, their liquid impermeability is enhanced by at least 10, at least 20, or at least 30 mbar, while WVTR values as stated before can be still achieved. Open materials which are not liquid-impermeable without coating but become more impermeable after coating are preferred.

If only one side of the leg cuffs is coated this is preferably the side facing the interior of the absorbent article. Preferably the leg cuffs are coated over the entire surface thereof. The configuration of the leg cuffs as such is not subject to any particular restrictions. Generally, it is however preferred to provide the absorbent article, in particular the diaper, panty diaper or incontinence device with elastically contractible gasketing cuffs each disposed adjacent to the two edges which extend in the longitudinal direction of the absorbent article and/or two barrier cuffs. The barrier cuffs are also arranged along the longitudinal edges of the absorbent article and are raised up from the topsheet. If two type of leg cuffs are used, the barrier cuffs are disposed inboard of said gasketing cuffs. Preferably spacing means are associated with said barrier cuffs for spacing its distal edge away from the top surface of the topsheet. Leg cuffs of this type and suitable materials (e.g. nonwoven webs) are disclosed for instance in US 4,695,278. An example for suitable barrier cuffs (here referred to as "raised edge barriers" is found in applicant's WO 01/66058.

According to one mode of this embodiment relating to treated leg cuffs, the liquid-permeable cover sheet is more hydrophilic in the central zone than in the end zones. In this connection, reference is made to the wording of claim 1 of WO 01/66058 describing this mode. If the "raised edge barriers" described in claim 1 and the remainder of this document are treated with a hydrophobic coating in accordance with the present invention, an extremely high leakage protection can be achieved.

According to one further embodiment of the present invention, the absorbent article comprises as the at least one further layer at least one fluid handling layer arranged between the coversheet and the absorbent layer. Said fluid handling layer comprises the coating of a hydrophobic thermoplastic polymer. Fluid handling layers, sometimes also referred to as "acquisition/distribution layers" are a common element of modern diapers and are included for quickly conducting incoming liquid away from the topsheet. Such structures are taught for instance by US 5,558,655, EP 0 640 330 A1, EP 0 631 768 A1, WO 95/01147 or WO 00/35502. They are typically effective in spreading the liquid in essentially parallel direction with respect to the topsheet surface to make optimum use of the absorbent capacity of the elongated absorbent layer (core). The present invention can be used for optimizing these fluid handling properties. The coating of thermoplastic polymer preferably covers the fluid handling layer only partially. The coating is preferably arranged in a pattern, one preferred arrangement involving parallel stripes running in the longitudinal direction of the fluid handling layer (i.e. the elongated direction of absorbent article). The stripes may have the same arrangement and size as already described in connection with partially coated topsheet materials. The same applies to the area coverage in percent.

Preferably the fluid handling layer is made from a hydrophilic foam, hydrophilic nonwoven or tissue paper sheet. Foam materials are well known in the art and for instance described in EP 0 878 481 A1 or EP 1 217 978 A1 in the name of the present applicant. Regarding the constitution of these materials reference can be made to the above description in connection with topsheet materials.

The partial coating of a hydrophobic thermoplastic polymer on the fluid handling layer, in particular its arrangement in parallel longitudinal stripes directs body fluids away from the insulting point thereby making better use of the absorbent capacity of the absorbent layer and preventing gel blocking. This effect can be further enhanced by the presence of hydrophilic fibers oriented in the longitudinal direction. US 4,676,786 illustrates for instance a fluid transport layer containing longitudinally oriented fluff pulp fibers which may be treated in accordance with the invention with the hydrophobic thermoplastic polymer.

The optional **absorbent layer** may comprise any absorbent material that is generally compressible, conformable, nonirritating to the wearer's skin and capable of absorbing and retaining liquids such as urine and other body exudates. The absorbent layer may be partially or totally surrounded by a core wrap. In some specific products it may also be totally omitted.

The absorbent layer may comprise a wide variety of liquid-absorbent materials commonly used in disposable diapers and other absorbent articles such as comminuted wood pulp, which is generally referred to as air felt or fluff. Examples of other suitable absorbent materials include creped cellulose wadding; melt blown polymers, including co-form; chemically stiffened, modified or cross-linked cellulosic fibers; tissue, including tissue wraps and tissue laminates, absorbent foams, absorbent sponges, superabsorbent polymers (such as superabsorbent fibers), absorbent gelling materials, or any other known absorbent materials or combinations of materials. Examples of some combinations of suitable absorbent materials are fluff with absorbent gelling materials and/or superabsorbent polymers, and absorbent gelling materials and superabsorbent fibers etc. The absorbent layer may also consist of two or more sublayers comprising one or more of the above absorbent materials.

The term "superabsorbent material" is well known in the art and designates water-swellable, water-insoluble materials (polymers, e.g. in fiber, flake or particle form) capable of absorbing the multiple of their own weight in body fluids. Preferably, the superabsorbent material is capable of absorbing at least about 10 times its weight, preferably at least about 15 times its weight, in particular at least about 20 times its weight in an aqueous solution containing 0.9 wt.-% of sodium chloride (under usual measuring conditions where the superabsorbent surface is freely accessible to the liquid to be absorbed). To determine the absorption capacity of the superabsorbent material, the standard test EDANA WSP 241.2 can be used.

Most preferably the absorbent article comprises cellulosic fluff fibers, optionally in combination with a superabsorbent material (SAP). If used in admixture, as frequently done in diapers, panty diapers or incontinence devices, the weight ratio of fluff based on the total mixture fluff/SAP is preferably 90 to 30 wt.-%, more preferably 80 to 35 wt.-%, in particular 70 to 40 wt.-%.

According to one further embodiment of the present invention, the preferably **liquid-impermeable coversheet** (**backsheet**) comprises the coating of a thermoplastic polymer. The backsheet typically prevents the exudates absorbed by the absorbent layer and containing with the article from soiling other external articles that may contact the absorbent article, such as bed sheets and undergarments. In preferred embodiments, the backsheet is substantially impermeable to liquids (e.g., urine) and comprises a laminate of a nonwoven and a thin plastic film such as a thermoplastic film having a thickness of about 0.012 mm to about 0.051 mm. Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the absorbent article while still preventing exudates from passing through the backsheet. Exemplary breathable materials may include materials such as woven webs, nonwoven webs, composite materials such as film-coated nonwoven webs, and microporous films. Since there is always a trade-off between breathability and liquid-impermeability it can be desired to provide backsheets showing a certain, relatively minor liquid-permeability but very high breathability values.

According to one mode of this embodiment, the backsheet is made from one layer of hydrophilic or hydrophobic nonwoven material (as described before) carrying the coating of hydrophobic thermoplastic polymer. This coating preferably extends over the entire surface of the nonwoven material. This mode may be conducted as follows.
- (A) Hydrophilic nonwovens of the type described before are at least partially (or fully) coated on at least one side, preferably both sides with the hydrophobic thermoplastic polymer. If one side is "partially" coated, the coating preferably constitutes from 5 to 90%, more preferably from 10 to 80%, in particular from 20 to 70% (e.g. from 30 to 60%) of the entire nonwoven area. The nonwoven to be coated contains at least some hydrophilic fibers. Regarding suitable amounts and types of hydrophilic fibers, reference can be made to the first embodiment (A) explained in connection with nonwovens for leg cuffs. According to one mode of the present embodiment, only those parts of the nonwoven that overlap with the absorbent layer (core) in the final absorbent article are coated with the thermoplastic polymer. It is preferred to use a precoated material, that is a material coated by nonwoven manufacturers and supplied as roll. Furthermore, it is preferred to apply the core in the right place in a process similar to that used to synchronize printed figures on the backsheet. For this purpose, a technique similar to the one disclosed in WO 00/45767 can be used. The thermoplastic polymer coating will prevent leakage of liquids from the core while still allowing some breathability through the region of the backsheet. Those areas of the backsheet which do not overlap with the core (or in more specific embodiments are not in contact with said absorbent core), for example the regions in the hip area or the belt of an incontinence product can remain uncoated. According to this embodiment, the hydrophilic fibers present in the nonwoven allow for an extremely high water vapour-permeability and thereby increased wearing comfort. The hydrophilic fibers can also absorb perspiration from the skin for improved comfort and skin health.
- (B) Hydrophobic liquid-impermeable nonwoven materials as currently used for backsheets and described before are coated on at least one side, preferably on both sides with the hydrophobic thermoplastic polymer to further enhance their liquid impermeability.
- (C) Rather lofty nonwoven materials which may not yet have the necessary liquid-impermeability are coated on at least one side, preferably both sides with the thermoplastic polymer to achieve an excellent balance of liquid impermeability and high breathability.

Regarding preferred liquid-impermeability values (and their increase) and/or breathability values of these three embodiments reference can be made to embodiments (A) to (C) explained in connection with nonwoven materials for leg cuffs.

The benefit of using hydrophilic nonwovens is that some regions can be left uncoated. It is also possible that a coated hydrophilic nonwoven (both sides) may be able to remove small liquid droplets from the skin regions where there is no core i.e. leg cuffs, hips, waist, belt.

According to one further mode, the preferably liquid impermeable cover sheet (**backsheet**) is a laminate material comprising at least two layers of a web or film material wherein said coating is present on at least one inner layer and/or the outer layer. "Inner" means in this context oriented towards the interior of the absorbent article and thus the skin of the wearer.

In embodiments, wherein the coating is present on at least one inner layer, this coated layer serves as hydrophobic barrier layer which allows for using materials having a higher breathability as outer layer. In this mode it is preferred to provide the coating on an inner layer (on the inner and/or the outer side of this "inner layer" of the backsheet) which is made from hydrophilic or hydrophobic nonwoven and to laminate the same to a microporous film as outer layer.

According to one alternative embodiment of the invention, the inner side of the outer layer (preferably a microporous film) of the backsheet laminate carries the hydrophobic coating.

According one further embodiment, the inner layer of the backsheet laminate is made of a microporous film and the outer layer preferably of nonwoven, woven or paper tissue whilst the inner and/or the inner side of said microporous film carries the coating.

The aforementioned microporous films can be made by producing a polymer film such as made from polyethylene, further comprising filler particles, such as calcium carbonate. After having formed a film wherein these filler particles are embedded into a matrix of the polymeric material the film is mechanically treated so as to strain and stretch the polymeric materials permanently thereby creating small cracks around the non-deforming filler particles. The cracks are sufficiently small to allow gas molecules of the gas phase to pass through but prevent liquids from penetrating. Such microporous films are available for instance from Mitsui Toatsu Co., Japan under the designation Espoire and are also described in US 4,705,812. The inner layer(s) is (are) preferably firmly bound to the outer layer although this is not essential to achieve the barrier layer effect.

In accordance with the present invention, the weight ratio of the coating of thermoplastic polymer to the uncoated layer (of web, foam or film material) preferably ranges from 1 to 25 wt.-%, more preferably 5 to 20 wt.-%, more preferably 3 to 20 wt.-%, in particular 5 to 15 wt.-%, based on the weight of the uncoated substrate material, i.e. the layer of web, foam or film material. If said layer is only partially coated, the above percentage values do not refer to the entire weight of said layer, but only to the weight of the coated areas.

The present invention also pertains to a method of making an absorbent article such as a diaper, panty diaper, panty liner, a sanitary napkin, an incontinence device or the like,
said article comprising at least a liquid-permeable topsheet made from at least one layer of web, foam or film material and a preferably liquid-impermeable backsheet made from at least one layer of web, foam or film material, and optionally at least one further layer of web, foam or film material, said method comprising the steps of
applying onto at least one of said layers a solution, preferably a hot solution of a hydrophobic thermoplastic polymer in an organic solvent and evaporating said solvent to form a coating having an irregular surface, and
incorporating said coated layer into the absorbent article.

According to one embodiment of the present invention, the absorbent article is obtainable by the above method, i.e. can be defined without reference to the contact angle of a sessile water drop. Nonetheless, according to preferred embodiments, the layer carrying the coating of thermoplastic polymer displays a contact angle of more than 110°, more preferably the previously disclosed values.

According to one embodiment of the claimed method, the layer to be coated is selected from hydrophilic or hydrophobic nonwovens, paper tissue or hydrophilic or hydrophobic plastic films. The present inventors have surprisingly found that layers made of hydrophilic or hydrophobic nonwovens are preferably fixed or stretched prior to the application of the hot solution of a hydrophobic thermoplastic polymer. The term "fixed" means in this context that at least one pair of parallel edges, preferably the entire circumference of the nonwovens (that is all four edges of square or rectangular nonwoven sheets) are fixed to a substrate, or within a frame or any other suitable device, without applying any forces that may extend the nonwoven parallel to its surface. The "stretching", on the other hand, can be performed in machine and/or cross direction of the nonwoven material, preferably in both directions. Depending on the type of nonwoven to be coated the stretching degree is preferably 0,1 to 30%, more preferably 0,5 to 10%, in particular 1 to 5%. The inventors have found that contacting nonwovens in a non-fixed or non-stretched state with a hot solution of hydrophobic thermoplastic polymer makes the nonwoven shrink after cooling and/or can generate cracks in the polymer coating.

According to one embodiment of the claimed method, the irregular surface of the thermoplastic polymer coating is generated by a technique described for the first time in the already mentioned Erbil reference. Accordingly, it is preferred to apply a hot solution of a thermoplastic polymer such as isotactic polypropylene (i-PP) in a volatile solvent followed by (relatively quick) evaporation of this solvent. Erbil describes in his article that the precipitation of isotactic polypropylene from a hot p-xylene solution leads to a porous morphology formed by a network of i-PP crystallites of different sizes and shapes and thereby an irregular surface.

Basically it is conceivable to apply a solution of the thermoplastic polymer in a volatile solvent having room temperature followed by evaporating the solvent under vacuum at room temperature.

However, it seems to be preferred to apply a hot solution of a thermoplastic polymer such as isotactic polypropylene to the layer to be coated. As "hot" we understand a solution heated above room temperature (25°C). A suitable temperature can be determined by a skilled person having regard to the nature of the solvent, the thermoplastic polymer and the layer material to be coated. Specifically, care should be taken that nonwoven or other layers do neither dissolve nor melt under the coating conditions and thereby collapse. Generally, it is preferred to apply a solution of thermoplastic polymer having a temperature of 30 to 170°C, e.g. 80 to 150°C, or 90 to 140°C.

Depending on the nature of the thermoplastic polymer a suitable solvent is selected. It can be selected from chlorinated aromatic and aliphatic hydrocarbons and preferably non-chlorinated aromatic and aliphatic hydrocarbons such as xylene or decalene. To ensure that the solvent is volatile enough it is moreover preferred to select solvents having a boiling point (at normal pressure of 1 atm) of 50 to 250°C, preferably 70 to 180°C, for instance 80 to 160°C or 100 to 150°C. Since boiling point and volatility do not necessarily correlate it is moreover preferred to select among the above solvents those having relatively low evaporation numbers. The evaporation number (Verdunstungszahl) can be determined according to the German industrial standard DIN 53170 under reference to the evaporation behaviour of diethyl ether (evaporation number = 1). Preferably, the selected solvent has an evaporation number of more than 1 but not greater than 35, in particular a number of 5 to 25, for instance 10 to 15 (xylol has an evaporation number of 13.5).

The (preferably hot) solution of thermoplastic polymer can be applied to the layer to be coated (web, for instance nonwoven or tissue paper sheet, film or foam) in various manners known in the art, for instance by dipping the layer into the solution, casting or printing the solution onto the layer or spraying the solution onto the layer.

After the application of the polymer solution the thermoplastic polymer can be deposited for instance as follows.
1) By subjecting the layer to which a hot polymer solution was applied to a spontaneous cooling in the ambient air.
2) By subjecting the layer to which a hot polymer solution was applied to a controlled cooling process, for instance by placing the layer in a drying oven having a lower temperature (for instance by at least 30 K, at least 40 K, at least 50 K, at least 60 K lower) than the temperature of the hot polymer solution followed by solvent evaporation in this drying oven. The drying oven may for instance have a temperature of 30 to 90°C, e.g. 50 to 80°C. To further enhance solvent evaporation a vacuum may be applied to the drying oven.
3) Any of the above processes may also be modified by adding a non-solvent for the thermoplastic polymer prior to solvent evaporation. Non-solvents for isotactic polypropylene are for instance acetone, dimethylformamide (DMF), methylethylketone (MEK), cyclohexanone or isopropyl alcohol. Erbil reports for instance that a superhydrophobic i-PP coating having a contact angle of 160° can be precipitated from a 60% p-xylene/40% MEK mixture by volume at an initial concentration of 20 mg/ml at 100°C followed by evaporating the solvent mixture at 70°C in a vacuum oven. The non-solvent shows preferably the same boiling point and/or evaporation number as given above for the solvent.

Drying can be performed by drying techniques known in the art such as hanging the treated substrates vertically or placing them horizontally in ambient air. In an industrial process it will be preferred to lead a continuous web after treating through a drying chamber. To accelerate solvent evaporation the layer, to which the polymer solution solvent was applied, can be exposed to a vacuum in each of the above modes (1) to (3).

From the results reported by Erbil it would appear that lower drying temperatures increase the contact angle. In a series of experiments with drying temperatures from 30 to 70°C higher contact angles were observed for the lower drying temperatures. Drying temperature can also be used to adjust the pore size and inhomogenity of the pores which apparently can be increased by longer crystallization time that is at lower drying temperatures.

The evaporation rate for the solvent may for instance be more than 50 weight-% of the solvent(s) in less than 5, less than 3 min or less than 1 min.

The present inventors have also found that polymer concentration influences the surface structure of the coating and correspondingly the contact angle. Generally, it is preferred to apply polymer solutions having a concentration of 1 to 100 mg/ml, more preferably 5 to 70 mg/ml, in particular 10 to 40 mg/ml.

### EXAMPLES

### Test procedures

### 1. Contact Angle

The contact angle was determined in line with TAPPI method T558PM-95 (1995) under consideration of the following:
i. The materials to be tested should be acclimatized at 23°C, 50% relative humidity over a suitable period of time (at least 4 h) prior to measurement. The measurement must be performed in a climate-controlled room (23°C, 50% relative humidity).
ii. The materials to be tested should be present as a single layer of material which can be applied to a standard sample holder using double sided adhesive tapes, as for instance recommended by the manufacture.
iii. Suitable parameters for the measurement are:
   a) liquid, reagent quality water
   b) a drop volume of 5 µl
   c) number of drops to be measured for averaging the results: 25
   d) in the hypothetical case where neither T558PM-95 nor the present comments address specific measurement conditions, default values as recommended by the manufacturer of the testing equipment can be used. Names of suppliers of suitable testing equipment may be found in the bound set of TAPPI test methods or may be availably from the TAPPI information resources centre. Preferred devices are manufactured by Fibro System AB, Stockholm and are marketed under the FibroDat^{®} Trademark, such as FibroDat 1100 contact angle tester.
iv. For those materials (e.g. hydrophilic, absorbent materials) where the contact angle varies with time, the measurement is conducted 0,05 sec after deposition of the drop.
v. If it is noted that the materials to be tested lead to very high contact angles, it may become necessary to adjust the force used for releasing the drop from the syringe to prevent the drop from rolling off.

### 2. ESEM (Environmental Scanning Electron Microscopy)

The electron microscope pictures were taken with a XL-30 TPM available from FEI company under standard conditions optimized for each individual sample.

### 3. Water impermeability

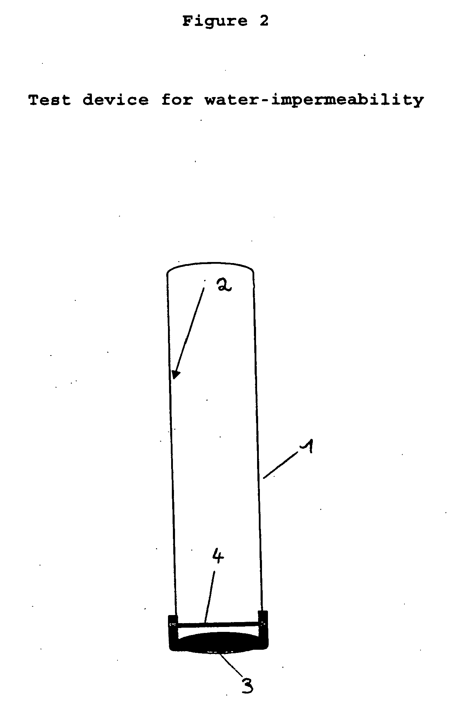

The water impermeability was roughly evaluated by measuring the hydrostatic pressure in a test device (1) as shown in Figure 2. Tap water was slowly put into the tube (pouring along the inner walls of the tube, for instance at a location shown by arrow (2), is recommended to minimize the impact of the running water on the nonwoven surface). The height of the liquid pillar on the tube was observed at the moment liquid began to drip through the nonwoven.

A more precise evaluation of water resistance is possible following the EDANA standard WSP 80.6 (05) using a 100 cm² test head, water having a temperature of 23 ± 2°C, a rate of increase of the water pressure of 10 ± 0,5 cm.

### 4. Water Vapour Transmission rate (WVTR)

### EDANA standard test WSP 70.6 (05) part 2

The vapour permeability was tested following EDANA standard test WSP 70.6 (05) part 2 with the coated substrates using a LYSSY-L80-4000 at 38°C.

### EXAMPLE 1 (Coating of web)

In a series of experiments an industrial wipe made from a blend of wood pulp, lyocell and polyester fibers was used as reference substrate for applying a strongly hydrophobic coating of isotactic polypropylene (i- PP). Isotactic polypropylene as being available from Sigma-Aldrich Co. under the product designation 182389 (10 mg/ml, 20 mg/ml or 30 mg/ml) and p-xylene (50 ml) were charged into a round bottom flask and the mixture was refluxed. The substrate was then coated with the hot solution in line with the following five coating techniques:
1. Dipping the substrate into the hot solution.
2. Casting the hot solution uniformly over the substrate.
3. About 50% of the hot solution is poured into a crystallization dish and then the substrate is placed into the bowl followed by pouring the rest of the solution over the substrate.
4. Putting the substrate into the round bottom flask together with the mixture of i-PP and p-xylene before heating, and
5. putting the substrate into the flask after dissolving the polypropylene and refluxing the mixture for a few minutes.

Furthermore, methylethylketone (MEK) was investigated as non-solvent in a series of experiments. For this purpose, i-PP (10 mg/ml, 20 mg/ml or 30 mg/ml) was dissolved in p-xylene (30 ml). After placing the substrate into the hot solution MEK (20 ml) was added. According to technique (1) the substrate was placed into the hot solution and then MEK (20 ml) was added. After removing the substrate the same was air-dried. It turned out that this specific variant of coating technique (1) using a p-xylene/MEK mixture is less suitable to achieve contact angles of more than 110°. Therefore, coating techniques (2) and (3) as set forth above were modified as follows:
A) dipping the substrate into MEK before coating,
B) dipping the substrate into MEK after coating.

The results are summarized in the following table.

**Table 1**

| Entry | Conc. PP | V P-xylene | V MEK | Coating Technique | DAT |
|---|---|---|---|---|---|
| 1 | 10 | 50 | | 2 | 111.6° |
| 2 | 10 | 50 | A | 3 | 117.7° |
| 3 | 20 | 50 | | 3 | 137.5° |
| 4 | 20 | 50 | | 2 | 132.3° |
| 5 | 20 | 50 | | 4 | 118.4° |
| 6 | 20 | 50 | | 5 | 130.9° |
| 7 | 20 | 50 | B | 2 | 127.4° |
| 8 | 30 | 50 | | 3 | 135.6° |
| Uncoated substrate | | | | | * |

| | | | | | |
|---|---|---|---|---|---|
| * The uncoated substrate absorbed the drops so quickly that contact angles could not be measured using DAT. | | | | | |

The coated wipes were evaluated by means of contact angle measurements (DAT = dynamic angle tester based on the sessile drop technique previously described) and ESEM (Environmental Scanning Election Microscopy).

ESEM pictures of one wipe coated in accordance with the present invention are shown in Figures 1 (A) and (B) in comparison to the uncoated substrate (Figure 1(C)). Further, the water-impermeability was evaluated using EDANA standard test WSP 80.6 (05), but a 10 cm² test head and 10cm/min pressure increase. The uncoated reference material absorbed water immediately so that hydrostatic pressure could not be measured. The coated sample showed a hydrostatic pressure value of 26 mbar (10 ± 0,5 cm H₂O/min).

Further, a vapour permeability test was conducted with the coated substrate using a LYSSY-L80-4000 in line with the test method described above. The vapour permeability measured was 2240 g/m² x 24 h.

### EXAMPLE 2 (Coating of tissue paper)

10 mg/ml of isotactic isopropylene (Sigma-Aldrich Co, 182389) were added to a mixture of 50 ml p-xylene/MEK in a volume ratio of 60/40 in a round bottom flask. The mixture was heated using an electric heating mantle until the i-PP granules disappeared. This could be achieved without refluxing the mixture. Then, one sheet of M-Tork^{®} hand drying single-ply tissue paper having a total basis weight of about 25 g/m² was coated in an area of about 100 cm² by pouring the hot solution uniformly over the paper. The sample was hung vertically to dry in ambient air. After about 12 hours, the sample was stored in a folded filter paper. After conditioning as described, the contact angle was measured in line with the previously described method to be 147°.

### EXAMPLE 3 (Coating of nonwoven)

60 ml p-xylene, 40 ml MEK and 2 g i-PP pellets (Sigma-Aldrich) were charged into a three neck round-bottom flask. One opening was fitted with a reflux condenser, one with a thermometer and the third one was closed with a glass stopper. The mixture was warmed using an electric heating mantle to a temperature of about 126°C. At this temperature the polypropylene was completely dissolved in p-xylene.

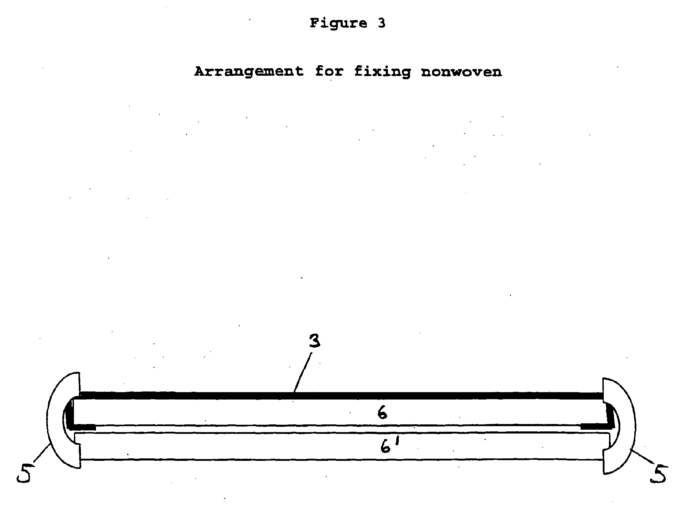

The nonwoven (3) to be coated (S1700PHW, a spunbond thermobonded hydrophobic 17 g/m² polypropylene nonwoven available from Union Industries SpA, Italy) was prepared by folding a sample over a glass plate (6) as shown in Fig. 3. A second glass plate (6') was placed under the first plate (4) in an arrangement as shown in Figure 3 to clamp the folded edges up against the first plate. The two glass plates were clamped together with clips (5). The purpose of this arrangement was to keep the nonwoven smooth and stretched during coating and cooling. The degree of stretching in both directions (MD/CD) was about 0,1 to 5%. However, any other arrangement suitable for an industrial process will equally work as long as it keeps the nonwoven under tension to prevent shrinking or wrinkling.

The nonwoven was coated using a metal lab spoon to cast small amounts of the hot polymer solution onto the nonwoven. The coated nonwoven was left hanging vertically to cool in ambient air and the solvents were allowed to evaporate in a fume hood at ambient temperature. The thoroughly cooled nonwoven was unwrapped from the glass plates and tested as follows.

A long glass tube (1) (as shown in figure 2) having a diameter of 1 cm was mounted vertically using clamps (not shown). The nonwoven to be tested was mounted over the bottom end of the glass tube by folding the nonwoven (3) up over the sides and securing the same with a rubber band (4). Care should be taken to avoid wrinkling in the area of the junction of the nonwoven with a glass wall of the tube. Tap water was slowly put into the tube (pouring along the inner walls of the tube is recommended to minimize the impact of the running water on the nonwoven surface). The height of the liquid pillar on the tube was observed at the moment liquid began to drip through the nonwoven. This method was not optimized for obtaining reliable absolute values but allowed a comparison with the uncoated nonwoven. Accordingly, it was observed that the uncoated nonwoven material began to leak at a water pillar height of about 1cm. In contrast thereto, the coated nonwoven did not begin to leak before the pillar had reached a height of several centimetres. This experiment thus clearly showed the increased liquid-impermeability of a nonwoven material coated in line with the present invention.

Such coated nonwoven can be used in absorbent products wherever, as previously explained, an increased hydrophobicity is beneficial, for instance as leg cuff material, as backsheet material or, if the corresponding coating is applied only partially, in particular in a pattern, as topsheet material or fluid handling layer.

## Claims

1. Absorbent article such as a diaper, panty diaper, panty liner, a sanitary napkin, an incontinence device or the like, comprising at least a liquid-permeable topsheet made from at least one layer of web, foam or film material and a backsheet made from at least one layer of web, foam or film material, and optionally at least one further layer of web, foam or film material,
wherein
(i) at least one of said layers comprises a coating of a hydrophobic thermoplastic polymer having an irregular surface and shows a contact angle of a sessile water drop of more than 110°, preferably more than 120°, more preferably more than 130°;
(ii) the hydrophobic thermoplastic polymer comprises crystalline domains; and
(iii) the irregular surface of the coating comprises individual deposits of coating material which may also aggregate and/or a network structure consisting of interconnected deposits forming pores.

2. Absorbent article according to claim 1 wherein the web material is selected from paper tissue and nonwoven.

3. Absorbent article according to claim 1 wherein the hydrophobic thermoplastic polymer is fluorine-free, preferably a polyolefin homo- or copolymer, more preferably isotactic polypropylene.

4. Absorbent article according to any of the preceding claims wherein the coating is non-sealing and preferably comprises pores wherein the majority of pores visible at the surface of the coating has a size below 100 µm.

5. Absorbent article according to any of the preceding claims wherein the coating is obtainable by contacting said layer with a solution of the thermoplastic polymer in an organic solvent and evaporating said solvent.

6. Absorbent article according to any of claims 1 to 5 wherein the coating of thermoplastic polymer is present in an amount of 1 to 25 weight-% based on the weight of coated substrate area.

7. Absorbent article according to any of the preceding claims wherein said liquid-permeable coversheet comprises said coating of a hydrophobic thermoplastic polymer, and is preferably made from a hydrophilic nonwoven or a hydrophilic perforated plastic film, wherein the coating of a thermoplastic polymer is preferably present in a pattern, wherein this pattern preferably comprises parallel stripes running in the longitudinal direction of the coversheet.

8. Absorbent article according to any of claim 1 to 7 wherein the absorbent articles comprises said at least one further layer, this layer comprise the coating of a hydrophobic thermoplastic polymer and is selected from
(i) leg cuffs and
(ii) a fluid-handling layer arranged between the coversheet and an absorbent layer, wherein this fluid-handling layer is preferably made from a hydrophilic nonwoven or a tissue paper sheet, and wherein the coating of the thermoplastic polymer is preferably present in a pattern, wherein this pattern preferably comprises parallel stripes running in the longitudinal direction of the fluid-handling layer.

9. Absorbent article according to any of claim 1 to 6 wherein the backsheet, preferably a liquid-impermeable backsheet comprises the coating of a thermoplastic polymer, and wherein the backsheet comprises a hydrophilic or hydrophobic nonwoven material carrying the coating.

10. Absorbent article according to claim 9 further comprising an absorbent layer wherein the nonwoven material is hydrophilic and the coating covers at least the area corresponding to the absorbent layer.

11. Absorbent article according to any of claim 1 to 6 wherein the backsheet is a laminate material comprising at least two layers of a web or film material and the coating is present on at least one inner layer and/or the outer layer.

12. Absorbent article according to claim 11 wherein one inner or the outermost layer is a microporous film.

13. Method of making an absorbent article such as a diaper, panty diaper, panty liner, a sanitary napkin, an incontinence device or the like,
the article comprising at least a liquid-permeable topsheet made from at least one layer of web, foam or film material and a backsheet made from at least one layer of web, foam or film material, and optionally at least one further layer of web, foam or film material, the method comprising the steps of
applying onto at least one of said layers a solution of a hydrophobic thermoplastic polymer in a solvent and evaporating the solvent to form a coating having an irregular surface, wherein (i) the coating of thermoplastic polymer having an irregular surface shows a contact angle of a sessile water drop of more than 110°, (ii) the hydrophobic thermoplastic polymer comprises crystalline domains, and (iii) the irregular surface of the coating comprises individual deposits of coating material which may also aggregate and/or a network structure consisting of interconnected deposits forming pores, and
incorporating the coated layer into the absorbent article.

14. Method according to claim 13 wherein the layer, onto which the solution is applied, is selected from hydrophilic or hydrophobic nonwovens, paper tissue or hydrophilic or hydrophobic plastic films.

15. Method according to claim 13 wherein the solution of thermoplastic polymer has a temperature above 25°C, preferably 80 to 150°C, when applied.

16. Method according to claim 15 wherein the layer to be coated is made of hydrophilic or hydrophobic nonwoven and said nonwoven is fixed or stretched prior to the application of the solution of a hydrophobic thermoplastic polymer.

## Patentansprüche

1. Absorptionsartikel, wie eine Windel, ein Windelhöschen, ein Höschenfutter, ein Sanitärtuch, eine Inkontinenzvorrichtung oder ähnliches, der mindestens folgendes umfasst: ein Flüssigkeits-durchlässiges Deckblatt, das aus mindestens einer Schicht aus Gewebe-, Schaum- oder Filmmaterial hergestellt ist und eine Rückschicht, die mindestens aus einer Schicht aus Gewebe-, Schaum- oder Filmmaterial hergestellt ist und wahlweise mindestens eine weitere Schicht aus Gewebe-, Schaum- oder Filmmaterial, worin
(i) mindestens eine dieser Schichten eine Beschichtung eines hydrophoben thermoplastischen Polymers umfasst, das eine unregelmäßige Oberfläche aufweist und einen Kontaktwinkel eines sessilen Wassertropfens von mehr als 110°, vorzugsweise mehr als 120°, besonders bevorzugt mehr als 130° zeigt;
(ii) das hydrophobe thermoplastische Polymer eine kristalline Domäne umfasst; und
(iii) die unregelmäßige Oberfläche der Beschichtung individuelle Ablagerungen des Beschichtungsmaterials umfasst, die ebenso aggregieren und/oder Poren einer Netzwerkstruktur bestehend aus miteinander verbundenen Ablagerungen bilden können.

2. Absorptionsartikel gemäß Anspruch 1, worin das Gewebematerial aus Papiertuch und -Fasern ausgewählt ist.

3. Absorptionsartikel gemäß Anspruch 1, worin das hydrophobe thermoplastische Polymer fluorfrei ist und vorzugsweise ein Polyolefin-Homo- oder Copolymer und besonders bevorzugt ein isotaktisches Polypropylen ist.

4. Absorptionsartikel gemäß mindestens einem der vorhergehenden Ansprüche, worin die Beschichtung nicht versiegelnd ist und vorzugsweise Poren umfasst, wobei die Mehrzahl der an der Beschichtungsoberfläche sichtbaren Poren eine Größe von unter 100 µm aufweist.

5. Absorptionsartikel gemäß mindestens einem der vorhergehenden Ansprüche, worin die Beschichtung erhältlich ist durch Kontaktieren der Schicht mit einer Lösung des thermoplastischen Polymers in einem organischen Lösungsmittel und Verdampfen des Lösungsmittels.

6. Absorptionsartikel gemäß mindestens einem der Ansprüche 1 bis 5, worin die Beschichtung des thermoplastischen Polymers in einer Menge von 1 bis 25 Gew.%, bezogen auf das Gewicht des beschichteten Subtratbereichs, vorliegt.

7. Absorptionsartikel gemäß mindestens einem der vorhergehenden Ansprüche, worin das Flüssigkeitsdurchlässige Deckblatt die Beschichtung eines hydrophoben thermoplastischen Polymers umfasst und vorzugsweise aus einer hydrophilen Faser oder einem hydrophilen perforierten Plastikfilmhergestellt ist, worin die Beschichtung eines thermoplastischen Polymers vorzugsweise in einem Muster vorliegt, wobei das Muster vorzugsweise parallele Streifen umfasst, die in der longitudinalen Richtung des Deckblatts verlaufen.

8. Absorptionsartikel gemäß mindestens einem der Ansprüche 1 bis 7, worin die Absorptionsartikel, die mindestens eine weitere Schicht umfassen, und diese Schicht die Beschichtung eines hydrophoben thermoplastischen Polymers umfasst, ausgewählt sind aus
(i) Beinmanschetten und
(ii) einer Flüssigkeits-Handhabungsschicht die zwischen der Deckschicht und einer Absorptionsschicht angeordnet ist, wobei diese Flüssigkeits-Handhabungsschicht vorzugsweise hergestellt ist aus einer hydrophilen Faser oder einem Papiertuchblatt und worin die Beschichtung des thermoplastischen Polymers vorzugsweise in Form eines Musters vorliegt, wobei dieses Muster vorzugsweise parallele Streifen umfasst, die in der longitudinalen Richtung der Flüssigkeits-Handhabungsschicht verlaufen.

9. Absorptionsartikel gemäß mindestens einem der Ansprüche 1 bis 6, worin die Rückschicht, vorzugsweise eine Flüssigkeits-undurchlässige Rückschicht, die Beschichtung eines thermoplastischen Polymers umfasst und wobei die Rückschicht ein hydrophiles oder hydrophobes Fasermaterial umfasst, das die Beschichtung trägt.

10. Absorptionsartikel gemäß Anspruch 9, der weiterhin eine Absorptionsschicht umfasst, wobei das Fasermaterial hydrophil ist und die Beschichtungsüberzüge mindestens den Bereich der Absorptionsschicht entsprechen.

11. Absorptionsartikel gemäß mindestens einem der Ansprüche 1 bis 6, worin die Rückschicht ein Laminatmaterial ist, welches mindestens zwei Schichten eines Gewebes oder Filmmaterials umfasst und die Beschichtung auf mindestens einer inneren Schicht und/oder der äußeren Schicht vorliegt.

12. Absorptionsartikel gemäß Anspruch 11, worin eine innere oder die äußerste Schicht ein mikroporöser Film ist.

13. Verfahren zur Herstellung eines Absorptionsartikel, wie einer Windel, einem Windelhöschen, einem Höschenfutter, einem Sanitärtuch, einer Inkontinenzvorrichtung oder ähnlichem,
der Artikel mindestens ein Flüssigkeits-durchlässiges Deckblatt umfasst, das aus mindestens einer Schicht eines Gewebe-, Schaum- oder Filmmaterials hergestellt ist, und ein Rückblatt, das mindestens aus einer Schicht eines Gewebe-, Schaum- oder Filmmaterials hergestellt ist und wahlweise mindestens eine weitere Schicht eines Gewebe-, Schaum- oder Filmmaterials umfasst, und das Verfahren die folgenden Schritte umfasst:
Anwenden einer Lösung eines hydrophoben thermoplastischen Polymers in einem Lösungsmittel auf mindestens einer dieser Schichten und Verdampfen des Lösungsmittels zur Bildung einer Beschichtung, die eine unregelmäßige Oberfläche aufweist, wobei (i) die Beschichtung des thermoplastischen Polymers eine unregelmäßige Oberfläche aufweist, die einen Kontaktwinkel eines sessilen Wassertropfens von mehr als 110° zeigt, (ii) das hydrophobe thermoplastische Polymer kristalline Domänen umfasst; und (iii) die unregelmäßige Oberfläche der Beschichtung individuelle Ablagerungen des Beschichtungsmaterials umfasst, die ebenso aggregieren und/oder Poren einer Netzwerkstruktur bestehend aus miteinander verbundenen Ablagerungen bilden können und
Einbringen der beschichteten Schicht in den Absorptionsartikel.

14. Verfahren gemäß Anspruch 13, wobei die Schicht, auf die die Lösung angewandt wird, ausgewählt ist aus hydrophilen oder hydrophoben Fasern, Papiertüchern oder hydrophilen oder hydrophoben Kunststofffilmen.

15. Verfahren gemäß Anspruch 13, wobei die Lösung des thermoplastischen Polymers eine Temperatur oberhalb von 25°C, vorzugsweise 80 bis 150°C aufweist, wenn sie angewandt wird.

16. Verfahren gemäß Anspruch 15, wobei die zu beschichtende Schicht aus hydrophilen oder hydrophoben Fasern hergestellt ist und die Fasern vor der Anwendung der Lösung des hydrophoben thermoplastischen Polymers fixiert oder gestreckt werden.

## Revendications

1. Article absorbant comme une couche, une couche-culotte, un protège-slip, une serviette hygiénique, un article pour incontinents ou un article similaire, comprenant au moins une feuille de dessus, perméable aux liquides et faite d'au moins une couche de matériau de type toile, mousse ou film, et une feuille de revers, faite d'au moins une couche de matériau de type toile, mousse ou film, ainsi que, en option, au moins une couche supplémentaire de matériau de type toile, mousse ou film, dans lequel article :
i) au moins l'une desdites couches comporte un revêtement de polymère thermoplastique hydrophobe et doté d'une surface irrégulière, et donne un angle de contact de goutte d'eau sessile de plus de 110°, de préférence de plus de 120° et mieux encore de plus de 130° ;
ii) le polymère thermoplastique hydrophobe comporte des domaines cristallins ;
iii) et la surface irrégulière du revêtement comporte des dépôts isolés de matériau de revêtement, qui peuvent aussi être agglomérés, et/ou une structure de type réseau, constituée de dépôts reliés entre eux et formant des pores.

2. Article absorbant conforme à la revendication 1, dans lequel le matériau de type toile est choisi parmi un papier à mouchoir et un non-tissé.

3. Article absorbant conforme à la revendication 1, dans lequel le polymère thermoplastique hydrophobe ne contient pas de fluor, et de préférence est un homopolymère ou un copolymère de type polyoléfine, et mieux encore, un polypropylène isotactique.

4. Article absorbant conforme à l'une des revendications précédentes, dans lequel le revêtement n'est pas étanche et comporte de préférence des pores parmi lesquels la majorité des pores visibles à la surface du revêtement ont une taille inférieure à 100 µm.

5. Article absorbant conforme à l'une des revendications précédentes, pour lequel on peut former le revêtement en mettant ladite couche en contact avec une solution du polymère thermoplastique dans un solvant organique, et en faisant s'évaporer ce solvant.

6. Article absorbant conforme à l'une des revendications 1 à 5, dans lequel le revêtement de polymère thermoplastique se trouve présent en une quantité représentant de 1 à 25 % du poids de la zone revêtue du substrat.

7. Article absorbant conforme à l'une des revendications précédentes, dans lequel c'est de ladite feuille de dessus perméable aux liquides que fait partie ledit revêtement de polymère thermoplastique hydrophobe, laquelle feuille est de préférence faite d'un non-tissé hydrophile ou d'un film perforé de plastique hydrophile, et dans lequel le revêtement de polymère thermoplastique se présente de préférence en un motif, lequel motif comprend de préférence des bandes parallèles disposées dans la direction longitudinale de la feuille de dessus.

8. Article absorbant conforme à l'une des revendications 1 à 7, lequel article absorbant comporte ladite couche supplémentaire au nombre d'au moins une, et c'est de cette couche que fait partie le revêtement de polymère thermoplastique hydrophobe, laquelle couche est choisie parmi
i) les serre-cuisse
ii) et une couche de rétention de fluide, disposée entre la feuille de dessus et une couche absorbante, laquelle couche de rétention de fluide est de préférence faite d'un non-tissé hydrophile ou d'une feuille de papier à mouchoir, le revêtement de polymère thermoplastique se présentant alors de préférence en un motif, lequel motif comprend de préférence des bandes parallèles disposées dans la direction longitudinale de la couche de rétention de fluide.

9. Article absorbant conforme à l'une des revendications 1 à 6, dans lequel c'est de la feuille de revers, de préférence une feuille de revers imperméable aux liquides, que fait partie le revêtement de polymère thermoplastique, et dans lequel la feuille de revers comprend un non-tissé hydrophile ou hydrophobe qui porte le revêtement.

10. Article absorbant conforme à la revendication 9, qui comprend en outre une couche absorbante, et dans lequel le matériau non-tissé est hydrophile et le revêtement couvre au moins la zone correspondant à la couche absorbante.

11. Article absorbant conforme à l'une des revendications 1 à 6, dans lequel la feuille de revers est faite d'un stratifié qui comporte au moins deux couches de matériau de type toile ou film, et le revêtement est placé sur au moins une couche interne et/ou la couche externe.

12. Article absorbant conforme à la revendication 11, dans lequel une couche interne ou la couche externe est un film microporeux.

13. Procédé de fabrication d'un article comme une couche, une couche-culotte, un protège-slip, une serviette hygiénique, un article pour incontinents ou un article similaire,
lequel article comprend au moins une feuille de dessus, perméable aux liquides et faite d'au moins une couche de matériau de type toile, mousse ou film, et une feuille de revers, faite d'au moins une couche de matériau de type toile, mousse ou film, ainsi que, en option, au moins une couche supplémentaire de matériau de type toile, mousse ou film, lequel procédé comporte les étape suivantes :
- étaler sur au moins l'une de ces couches une solution d'un polymère thermoplastique hydrophobe dans un solvant, et faire s'évaporer le solvant de manière à former un revêtement doté d'une surface irrégulière,
i) lequel revêtement fait d'un polymère thermoplastique et doté d'une surface irrégulière donne un angle de contact de goutte d'eau sessile de plus de 110°,
ii) dans lequel revêtement le polymère thermoplastique hydrophobe comporte des domaines cristallins,
iii) et la surface irrégulière duquel revêtement comporte des dépôts isolés de matériau de revêtement, qui peuvent aussi être agglomérés, et/ou une structure de type réseau, constituée de dépôts reliés entre eux et formant des pores ;
- et incorporer la couche ainsi revêtue dans l'article absorbant.

14. Procédé conforme à la revendication 13, dans lequel la couche sur laquelle la solution est étalée est choisie parmi un non-tissé hydrophile ou hydrophobe, un papier à mouchoir ou un film de plastique hydrophile ou hydrophobe.

15. Procédé conforme à la revendication 13, dans lequel la solution de polymère thermoplastique se trouve, lors de son étalement, à une température supérieure à 25 °C, et de préférence de 80 à 150 °C.

16. Procédé conforme à la revendication 15, dans lequel la couche à revêtir est faite d'un non-tissé hydrophile ou hydrophobe, et l'on fixe ou l'on étire ce non-tissé avant d'étaler dessus la solution de polymère thermoplastique hydrophobe.
